Europäisches Patentamt

European Patent Office

Office européen des brevets

(11) Veröffentlichungsnummer: **0 303 104**
**B1**

(12) **EUROPÄISCHE PATENTSCHRIFT**

(45) Veröffentlichungstag der Patentschrift: **16.01.91**

(51) Int. Cl.⁵: **A 61 L 15/18, A 61 F 13/02**

(21) Anmeldenummer: **88112096.8**

(22) Anmeldetag: **27.07.88**

(54) Wundabdeckung.

(30) Priorität: **11.08.87 DE 3726617**

(43) Veröffentlichungstag der Anmeldung:
**15.02.89 Patentblatt 89/07**

(45) Bekanntmachung des Hinweises auf die
Patenterteilung:
**16.01.91 Patentblatt 91/03**

(84) Benannte Vertragsstaaten:
**AT BE CH DE ES FR GB GR IT LI LU NL SE**

(56) Entgegenhaltungen:
**EP-A-0 188 942**
**DE-A-2 305 989**
**DE-A-2 412 128**

(73) Patentinhaber: **Friedrichsfeld GmbH Keramik-
und Kunststoffwerke
Steinzeugstrasse 50
D-6800 Mannheim 71 (DE)**

(72) Erfinder: **Meiss, Ludwig, Dr. med.
Reichskanzlerstrasse 21a
D-2000 Hamburg (DE)**

(74) Vertreter: **Klose, Hans, Dipl.-Phys. et al
Kurfürstenstrasse 32
D-6700 Ludwigshafen (DE)**

Courier Press, Leamington Spa, England.

**Beschreibung**

Wundabdeckungen werden bekanntlich für Brandwunden, großflächige Hautwunden oder Hautdefekte, offene Weichteilverletzungen, Druckgeschwüre (Decubitus), sogenannte "offene Beine" (Ulcera cruris), Wundaufbrüche (Wunddehiszenzen) und dergleichen benötigt. Derartige Wunden sind einer Kontamination durch Keime (einschließlich Pilze) ausgesetzt und können dann zu septischen Wunden werden, die schlecht heilen und eine spezielle Wundabdeckung und Wundbehandlung erfordern.

Für derartige Anwendungsfälle gibt es bisher Mullkompressen sowie Kompressen aus anderen geeigneten Materialien, wobei organische Materialien, insbesondere Schweinehaut oder Collagenvlies, oder Metallfolien, wie insbesondere Aluminiumfolien, zum Einsatz gelangen. Ferner können synthetische organische Materialien in Form von Schäumen, Gelen oder Filmen, Matten und Pulvern benutzt werden. Hierzu zählen Fettgaze, Polyurethanschäume und -matten, Abdeckungen aus Polytetrafluorid oder Polyvinylchlorid, Teflon-Polyurethan-Folien, Materialien auf Silikonbasis sowie flüssigkeitsabsorbierende Pulver (z.B. auf Dextranbasis). Die derzeit bekannten Wundabdeckungen aus den genannten Materialien werden meist in Form von Platten oder Matten auf die Wunden gelegt. Hierbei kann es mitunter zur Verhaltung von Wundsekret unter dem benutzten Abdeckungsmaterial kommen und nachfolgend zu einer Verzögerung der Wundheilung. Bei Vorliegen einer Keimbesiedlung resultiert ein weiterschwelender Infekt, und zwar unter Umständen auch bei einem mehrfachen täglichen Wechsel des Verbandes.

Aus der DE—A—11 61 384 ist ein metallisierter, saugfähiger Verbandstoff bekannt. Dieser Verbandstoff enthält ein feinporiges, saugfähiges, filzartig verdichtetes Faservlies, das auf den Faseroberflächen ohne Beeinträchtigung der Vliesstruktur mit einem feinen Metallüberzug versehen ist. Derartige metallisierte Verbandstoffe vermitteln keine Anregung, eine Schicht aus Keramik oder Glas im Sinne der vorliegenden Erfindung vorzusehen, um die nachfolgend zu beschreibenden Vorteile und Wirkungen zu erhalten.

Ferner ist aus der DE—A—24 12 128 ein Material zur Bildung eines chirurgischen Verbandes und ein Verfahren zur Bildung desselben in situ nachgewiesen, welches aus inerten keramischen Mikroteilchen besteht. Diese Mikroteilchen weisen ein spezifisches Gewicht über 1 und ferner eine Größe im Bereich der Siebzahl 100 bis 3000 auf. Der vorbekannte chirurgische Verband wird auf der Stelle durch Auflegen der genannten keramischen Mikroteilchen unmittelbar auf die nassen Gewebeoberflächen in der Verletzungszone hergestellt. Die Mikroteilchen können ähnlich Puder auf die Wunde aufgestäubt werden. Eine Verbindung mittels Verbindungselementen oder einem Vlies ist das Gegenteil zur vorbekannten Bildung eines chirurgischen Verbandes in situ.

Für derartige Wundabdeckungen besteht vor allem die Forderung nach Permeabilität für Sauerstoff und Flüssigkeiten (z.B. Wundsekrete und therapeutische Lösungen). Ferner sollen derartige Abdeckungen gewebefreundlich ebenso wie granulationsstimulierend sein, also biokompatibel und möglichst auch bioaktiv beschaffen sein. Darüber hinaus besteht die Forderung nach hinreichender mechanischer Flexibilität. Die genannten Forderungen werden mit den derzeit bekannten Materialien nicht in zufriedenstellender Weise erfüllt, wobei regelmäßig nur einzelne Forderungen und nicht alle gleichzeitig erfüllt werden.

Die Lösung dieser Aufgabe erfolgt gemäß den im Kennzeichen des Patentanspruchs 1 angegebenen Merkmalen.

Die vorgeschlagene Wundabdeckung gewährleistet einen guten Kontakt mit dem Wundgrund infolge der Gewebefreundlichkeit und Durchtrittsmöglichkeit von Sekret, besonders auch von keimhaltigem Sekret, d.h. Eiter. Mit der Wundabdeckung kann zuverlässig die Heilung verbessert werden und eine verbesserte Ausgangssituation für den Fall einer Hauttransplantation geschaffen werden. Durch die erfindungsgemäß vorgeschlagene Art der Oberfläche, sei es von zu einem Netzwerk verbundenen Körpern oder von einem beschichteten Grundnetz, wird überraschend die Bildung von sauberem Granulationsgewebe gefördert. Es hat sich herausgestellt, daß bei Anwendung der erfindungsgemäßen Wundabdeckung die Intervalle für einen Verbandswechsel im Vergleich mit vorbekannten Wundabdeckungen nicht unwesentlich verlängert werden können. Die zu einem Netzwerk verbundenen Körper weisen ebenso wie das beschichtete Grundnetz eine gute Flexibilität auf und lassen sich problemlos an die Kontur der abzudeckenden Wunde anpassen.

Die genannten Körper sind zumindest an ihrer Oberfläche in der erfindungsgemäßen Weise mit einer Schicht aus Keramik oder Glas versehen, wobei im Rahmen dieser Erfindung die Körper auch gänzlich aus genannten Materialien hergestellt sein können. Die Körper können zur Herstellung des Netzwerkes mit Löchern oder durchgehenden Bohrungen versehen sein, wobei mittels durchgezogenen Fäden ein ein- oder mehrdimensionales Netzwerk aufgebaut wird. Die Körper können mittels spezieller Verbindungselemente oder Verfahren zu einem ein- oder mehrdimensionalen Netzwerk verbunden werden, wobei sie in ihrer Größe und Form den Erfordernissen entsprechend ausgebildet sind. Die Verbindungselemente können ferner unmittelbar an den genannten Körpern angeordnet sein und in Form von Haken und Ösen, Nut und Feder, Schnappverschlüssen oder in ähnlicher Weise die gegenseitige Verbindung der einzelnen Körper bewirken.

In der alternativen Ausführungsform wird ein nachgiebiges, flexibles Grundnetz, welches insbesondere als eine Matte, Vlies oder Gewebe ausgebildet ist, mit einer Schicht aus Keramik oder Glas versehen. Derartige Grundnetze können aus Metall, Keramik oder aus organischem Gewebe bestehen. Die Beschichtung kann in einfacher Weise und zuverlässig aufgrund bekannter Technologien entstehen, wobei an dieser Stelle insbesondere thermische Spritzverfahren, Elektrophorese sowie CVD- und PVD- Verfahren (chemical bzw. physical vapour deposition) zum Einsatz gelangen können.

Die vorgeschlagene Wundabdeckung entspricht in vereinheitlichter Form den oben aufgezeigten Anforderungen und weist eine hervorragende Permeabilität für Sauerstoff und Flüssigkeiten auf, sie ist gewebefreundlich und granulationsstimulierend und zeichnet sich durch eine gute mechanische Flexibilität aus.

An dieser Stelle sei ausdrücklich festgehalten, daß die Wundabdeckung nicht aus identischen, einzeln übereinstimmenden Körpern zusammengesetzt sein muß. Vielmehr können im Rahmen dieser Erfindung für eine Wundabdeckung jeweils auch Körper unterschiedlicher Geometrie und Größe zum Einsatz gelangen. Die Körper können als Platten, Rohre, Kugeln oder auch als Ringe ausgebildet sein, die in ihrer jeweiligen projezierten Fläche ein Bruchteil der zu bedeckenden Wundfläche ausmachen. In einer besonderen Ausführungsform können die Körper mit Antibiotika und/oder antiseptischen (antibakteriellen, antiviralen, antimykotischen) Substanzen versehen sein. Schließlich können auch die Körper oder das für die Beschichtung zum Einsatz gelangende Material selbst bzw. die Verbindungselemente oder das Grundnetz eine antiseptische Wirkung im Rahmen der Erfindung aufweisen. Es hat sich gezeigt, daß Silberverbindungen eine derartige Wirkung aufweisen, und so kann insbesondere die zum Einsatz gelangende Keramik mit einer derartigen Silberverbindung erfindungsgemäß versetzt sein.

Weitere wesentliche Merkmale und Vorteile ergeben sich aus den Unteransprüchen sowie der nachfolgenden Beschreibung von Ausführungsbeispielen. Es zeigen:

Figur 1 einen einzelnen quadratisch ausgebildeten Körper,

Figur 2 ausschnittsweise eine Wundabdeckung aus mehreren quadratischen Körpern gemäß Figur 1,

Figur 3 teilweise eine Wundabdeckung mit länglichen Körpern,

Figur 4, 5 zylindrische Körper mit zwei bzw. vier Bohrungen zum Durchziehen von Fäden,

Figur 6 einen Längsschnitt durch einen zylindrischen Körper mit abgerundeten Stirnflächen,

Figur 7 einen Schnitt durch einen kugelförmigen Körper,

Figur 8 Körper mit ineinandergreifenden Haken bzw. Ösen.

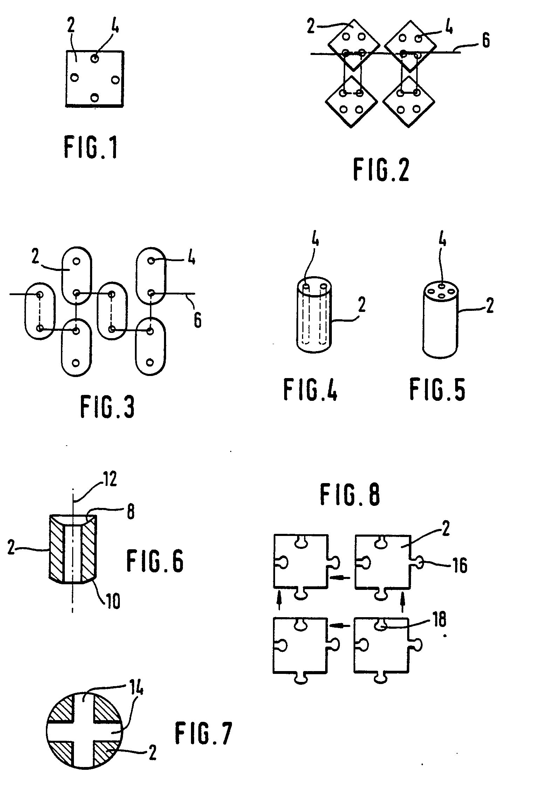

In Figur 1 ist ein kleiner quadratischer Körper 2 dargestellt, und zwar in etwa doppelter bis fünffacher natürlicher Größe. Der Körper 2 könnte auch eine andere Außenkontur aufweisen, beispielsweise rechteckförmig, rautenförmig, rund oder dergleichen ausgebildet sein. Die Dicke dieses Körpers 2, also senkrecht zur Zeichnungsebene gesehen, beträgt ca. ein bis mehrere Millimeter. Dieser Körper 2 weist vier Bohrungen 4 auf, durch welche zur Herstellung der Verbindung mit weiteren entsprechenden Körpern ein oder auch mehrere Fäden hindurchgeführt werden.

Figur 2 zeigt in einer Aufsicht einen Teil einer Wundabdeckung, welche aus einer Vielzahl von quadratischen Körpern 2 zu einem Netzwerk zusammengesetzt ist. Zur Verbindung dieser plattenförmigen Körper 2 ist ein Faden 6 vorgesehen, welcher in der dargestellten Weise durch die Bohrungen 4 von einander benachbarten Körpern 2 hindurchgefädelt ist. Es ist ersichtlich, daß mit weiteren Fäden in entsprechender Weise die Verbindung mit den anderen plattenförmigen Körpern 2 erfolgen kann. Wesentlich ist, daß mit den hier beispielschaft aus Fäden bestehenden Verbindungselementen eine Vielzahl von kleinen Körpern 2 miteinander verbunden ist, wobei diese Körper zumindest an der Oberfläche eine Schicht aus Keramik oder Glas aufweisen. Selbstverständlich können die Körper auch vollständig aus einem derartigen Material bestehen.

Figur 3 zeigt wiederum nur teilweise eine Wundabdeckung, deren Körper 2 nunmehr eine langgestreckte oder ovale Form aufweisen. Auch diese Körper 2 sind im wesentlichen plattenförmig ausgebildet, wobei die Materialdicke im Millimeterbereich liegt. Zur Herstellung der gegenseitigen Verbindung weisen diese Körper 2 jeweils zwei Bohrungen 4 auf, durch welche Fäden hindurchgeführt werden können.

Figur 4 und 5 zeigen Ausführungsformen der Körper, welche im wesentlichen zylinderförmig ausgebildet sind und zwei bzw. vier Längsbohrungen 4 aufweisen. Derartige zylindrische Körper 2 können in axialer Richtung hintereinander, ähnlich einer Kette mittels Fäden miteinander verbunden werden; eine Vielzahl derartiger "Ketten" sind nebeneinander angeordnet und bilden in ihrer Gesamtheit die Wundabdeckung.

Figur 6 zeigt eine zweckmäßige Ausgestaltung des wiederum zylindrisch ausgebildeten Körpers 2. Wie in diesem axialen Längsschnitt ersichtlich, ist die obere Stirnfläche 8 konkav und die untere Stirnfläche 10 konvex ausgebildet. In der Richtung der Längsachse 12 sind derartige Körper 2 hintereinander angeordnet, wobei von benachbarten Körpern die konvexen Stirnflächen entsprechend in der konkaven Stirnfläche gelagert sind. Auch hier lassen sich problemlos nebeneinander eine Vielzahl derartiger "Ketten" mittels Verbindungselementen in Form von Fäden oder dergleichen vorsehen.

Figur 7 zeigt eine weitere Ausführungsform des Körpers, der hier kugelförmig ausgebildet ist. Wie ersichtlich, sind zwei zueinander orthogonale Bohrungen 14 vorhanden, durch welche zur Herstellung einer kompletten Wundabdeckung in entsprechender Weise Verbindungselemente in Form von Fäden durchgezogen werden können.

In Figur 8 ist eine Ausführungsform dargestellt, deren Körper 2 ineinander greifende Haken 16 und Ösen 18 aufweisen. Die im wesentlichen quadratisch ausgebildeten Platten 2 weisen an zwei Längsseiten zwei derartige Haken 16 und an den beiden anderen Seiten zwei Ösen 18 in der dargestellten Weise auf. Die Haken und Ösen können nach Art von Schnappverschlüssen ausgebildet sein, damit bei einfacher Herstellung gleichwohl eine zuverlässige Verbindung sichergestellt wird. Um eine gute Flexibilität und Beweglichkeit der einzelnen Körper 2 untereinander zu gewährleisten, sind die Haken 16 (halb)kugelähnlich ausgebildet und mit etwas Spiel in die ebenfalls abgerundet ausgebildeten Ösen eingesetzt.

Bezugszeichenliste
2      Körper
4      Bohrung
6      Faden
8      konkave Stirnfläche
10    konvexe Stirnfläche
12    Längsachse
14    Längsbohrung
16    Haken
18    Öse

## Patentansprüche

1. Wundabdeckung, dadurch gekennzeichnet, daß mittels Verbindungselementen (6; 16, 18) zu einem Netzwerk verbundene Körper (2) zumindest an der Oberfläche eine Schicht aus Keramik oder Glas aufweisen oder daß ein Grundnetz mit einer derartigen Schicht versehen ist.

2. Wundabdeckung nach Anspruch 1, dadurch gekennzeichnet, daß das Grundnetz als eine Matte, ein Vlies oder ein Gewebe ausgebildet ist.

3. Wundabdeckung nach Anspruch 1, dadurch gekennzeichnet, daß die Körper (2) vollständig aus einem bioinerten Werkstoff, vorzugsweise aus Aluminiumoxid, $Al_2O_3$, bestehen.

4. Wundabdeckung nach Anspruch 1, dadurch gekennzeichnet, daß die Körper (2) vollständig aus einem bioaktiven Werkstoff bestehen.

5. Wundabdeckung nach Anspruch 1, dadurch gekennzeichnet, daß die Körper (2) gleichzeitig aus bioinerten und bioaktiven Werkstoffen bestehen.

6. Wundabdeckung nach Anspruch 1, dadurch gekennzeichnet, daß die Körper (2) aus einem nichtkeramischen Kern und einer äusseren Schicht aus Keramik oder Glas bestehen.

7. Wundabdeckung nach einem der Ansprüche 1 bis 6, dadurch gekennzeichnet, daß die Verbindungselemente oder das Grundnetz aus Metall oder nichtmetallischem anorganischen Material bestehen.

8. Wundabdeckung nach einem der Ansprüche 1 bis 6, dadurch gekennzeichnet, daß die Verbindungselemente oder das Grundnetz aus einem organischen Material bestehen.

9. Wundabdeckung nach einem der Ansprüche 1, 3 bis 8, dadurch gekennzeichnet, daß die jeweils projizierte Fläche der Körper (2) einen Bruchteil der zu bedeckenden Wundfläche beträgt.

10. Wundabdeckung nach Anspruch 9, dadurch gekennzeichnet, daß die Körper (2) die Form von Platten, Kugeln, Rohren, Ringen aufweisen.

11. Wundabdeckung nach einem der Ansprüche 4 bis 10, dadurch gekennzeichnet, daß der bioaktive Werkstoff aus Calciumphosphaten, vorzugsweise aus Tricalciumphosphat und/oder Hydroxylapatit und/oder aus einem Bioglas und/oder einer Bioglaskeramik oder aus einem Gemisch dieser Werkstoffe besteht.

12. Wandabdeckung nach einem der Ansprüche 1 bis 11, dadurch gekennzeichnet, daß die Körper (2) aus einem flüssigkeitsdichten Werkstoff bestehen.

13. Wundabdeckung nach einem der Ansprüche 1 bis 11, dadurch gekennzeichnet, daß die Körper (2) eine Porosität aufweisen.

14. Wundabdeckung nach Anspruch 13, dadurch gekennzeichnet, daß die Körper mit Antibiotika und/oder antiseptischen, insbesondere antibakteriellen, antiviralen und antimykotischen, Substanzen versehen sind.

15. Wundabdeckung nach einem der Ansprüche 1 bis 14, dadurch gekennzeichnet, daß der Werkstoff der Körper, der Schicht und/oder der Verbindungselemente oder des Grundnetzes eine antiseptische Wirkung aufweist.

16. Wundabdeckung nach Anspruch 15, dadurch gekennzeichnet, daß der genannte Werkstoff mit einer Silberverbindung vernetzt ist.

## Revendications

1. Pansement pour plaies, caractérisé en ce que des pièces (2), qui sont assemblées en un réticule à l'aide d'éléments d'assemblage (6, 16, 18), présentent, du moins sur la surface, une couche de céramique ou de verre ou en ce qu'un réticule de base est muni d'une telle couche.

2. Pansement pour plaies selon la Revendication 1, caractérisé en ce que le réticule de base est constitué par un feutre, un non tissé ou un tissu.

3. Pansement pour plaies selon la Revendication 1, caractérisé en ce que les pièces (2) sont entièrement composées d'une matière bio-inerte, de préférence d'oxyde d'aluminium, $Al_2O_3$.

4. Pansement pour plaies selon la Revendication 1, caractérisé en ce que les pièces (2) sont entièrement composées d'une matière bio-active.

5. Pansement pour plaies selon la Revendication 1, caractérisé en ce que les pièces (2) sont composées à la fois de matières bio-inertes et de matières bioactives.

6. Pansement pour plaies selon la Revendication 1, caractérisé en ce que les pièces (2) sont composées d'un noyau non céramique et d'une couche extérieure en céramique ou en verre.

7. Pansement pour plaies selon une des Revendications 1 à 6, caractérisé en ce que les éléments d'assemblage ou le réticule de base est fait de métal ou d'une matière inorganique non métallique.

8. Pansement pour plaies selon une des Revendications 1 à 6, caractérisé en ce que les éléments d'assemblage ou le réticule de base sont composés d'une matière organique.

9. Pansement pour plaies selon une des Revendications 1, 3 à 8, caractérisé en ce que la surface projetée des pièces (2) représente une fraction de la surface de plaie à recouvrir.

10. Pansement pour plaies selon la Revendication 9, caractérisé en ce que les pièces (2) présentent la forme de plaquettes, de sphères, de tubes ou d'anneaux.

11. Pansement pour plaies selon une des Revendications 4 à 10, caractérisé en ce que la matière bio-active est composée de phosphates de calcium, de préférence de phosphate tricalcique et/ou d'hydroxylapatite et/ou d'un bioverre et/ou d'une biocéramique vitreuse ou d'un mélange de ces matières.

12. Pansement pour plaies selon une des Revendications 1 à 11, caractérisé en ce que les pièces (2) sont composées d'une matière imperméable aux liquides.

13. Pansement pour plaies selon une des Revendications 1 à 11, caractérisé en ce que les pièces (2) présentent une certaine porosité.

14. Pansement pour plaies selon la Revendication 13, caractérisé en ce que les pièces sont munies d'antibiotiques et/ou de substances antiseptiques, notamment bactéricides, antivirales et antimycosiques.

15. Pansement pour plaies selon une des Revendications 1 à 14, caractérisé en ce que la matière des pièces, de la couche et/ou des éléments d'assemblage ou du réticule de base ont une action antiseptique.

16. Pansement pour plaies selon la Revendication 15, caractérisé en ce que la matière précitée est imprégnée d'un composé d'argent.

## Claims

1. Wound covering, characterised in that elements (2) having at least on the surface a layer of ceramic or glass are joined together by connecting members (6; 16, 18) to form a network or in that a base network is provided with a layer of this kind.

2. Wound covering according to Claim 1, characterised in that the base network is in the form of a mat, a non-woven material or a textile fabric.

3. Wound covering according to Claim 1, characterised in that the elements (2) consist entirely of a bioinert material, preferably aluminium oxide, $Al_2O_3$.

4. Wound covering according to Claim 1, characterised in that the elements (2) consist entirely of a bioactive material.

5. Wound covering according to Claim 1, characterised in that the elements (2) consist of bioinert and bioactive materials at the same time.

6. Wound covering according to Claim 1, characterised in that the elements (2) consist of a non-ceramic core and an outer layer of ceramic or glass.

7. Wound covering according to one of Claims 1 to 6, characterised in that the connecting elements or the base network consist of metal or non-metallic inorganic material.

8. Wound covering according to one of Claims 1 to 6, characterised in that the connecting elements or the base network consist of an organic material.

9. Wound covering according to one of Claims 1, 3 to 8, characterised in that the projected surface of the elements (2) amounts to a fraction of the wound surface which is to be covered.

10. Wound covering according to Claim 9, characterised in that the elements (2) are in the form of plates, spheres, tubes or rings.

11. Wound covering according to one of Claims 4 to 10, characterised in that the bioactive material consists of calcium phosphates, preferably tricalcium phosphate and/or hydroxyl apatite and/or a bioglass and/or a bioglass ceramic or a mixture of these materials.

12. Wound covering according to one of Claims 1 to 11, characterised in that the elements (2) consist of a fluid-impervious material.

13. Wound covering according to one of Claims 1 to 11, characterised in that the elements (2) are porous.

14. Wound covering according to Claim 13, characterised in that the elements are provided with antibiotics and/or antiseptic, more particularly antibacterial, antiviral and antimycotic substances.

15. Wound covering according to one of Claims 1 to 14, characterised in that the material of the elements, the layer and/or the connecting elements or the base network has an antiseptic activity.

16. Wound covering according to Claim 15, characterised in that the material specified is treated with a silver compound.

FIG.1

FIG.2

FIG.3

FIG.4

FIG.5

FIG.8

FIG.6

FIG.7